# EUROPEAN PATENT APPLICATION

(11) **EP 2 460 894 A1**
(43) Date of publication of application: **06.06.2012**
(21) Application number: 11192254.8
(22) Date of filing: 06.12.2011
(51) Int. Cl.: C12Q 1/68

(54) **Prognostic marker for mamma carcinoma**

(30) Priority: 06.12.2010 EP 10193883; 07.12.2010 EP 10194061
(71) Applicant: Medizinische Hochschule Hannover, 30625 Hannover (DE)
(72) Inventor: Dörk-Bousset, Thilo, 30629 Hannover (DE); Heuser, Michael, 30655 Hannover (DE); Damm, Frederik Martin Georges, 75014 Paris (DE); Ganser, Arnold, 30559 Hannover (DE); Park-Simon, Tjoung-Won, 30559 Hannover (DE); Hillemanns, Peter, 30625 Hannover (DE)
(74) Representative: Taruttis, Stefan Georg

(57) **Abstract**

The invention provides an analytical method for the determination of the susceptibility of a human patient suffering from breast cancer to anti-cancer treatment by providing an analytical method for determining the genetic predisposition of a patient in respect of susceptibility, e.g. responsiveness, to anti-cancer treatment, especially treatment using at least one chemotherapeutical agent and/or at least one anti-hormonal agent on the basis of analysis of a genetic marker.

## Description

The invention relates to the analysis of a genetic marker for use as a prognostic marker in breast cancer, and to the analysis, and to the genetic marker, respectively, as an analyte for use in the selection of a therapy, as well as to an analytical method identifying the marker in breast cancer patients, especially for use in the prediction of the suitability of a treatment for a selected sub-group of patients. Further, the invention relates to the use of therapeutic agents for the long-term treatment, e.g. in a period of at least 3 to 5 years following initial cancer treatment or cancer diagnosis, especially in a period of at least 4 to 10 years following initial cancer treatment or cancer diagnosis, to improve the long-term treatment of breast cancer in a selected sub-group of patients, who are at least heterozygous for the genetic marker. The invention is based on the finding that in lobular breast cancer patients, one allele of the genetic marker is correlated with a poorer treatment prognosis, and hence the sub-group of patients requiring the administration of pharmaceutically active compounds as a medicament for treatment and/or prevention of lobular breast cancer, especially as a medicament for the prevention of recurring lobular breast cancer is characterized by the at least heterozygous presence of one allele of the marker. Interestingly, both alleles of the marker used in the invention give the same translation product, making the alleles a silent mutation.

Hollink et al., Blood 5951-5960 (2009) found that there is no relation of the status of the marker Ref SNP ID rs16754 to the course of cancer in patients, because the alleles of the marker Ref SNP ID rs16754 have no influence on the amino acid sequence of the protein produced from the WT1 gene concerned.

Li and Man, Cancer Biomark. 109-116 (2009) describe that the expression of the WT1 gene is changed in breast cancer cells. Miyoshi et al., Clin. Cancer Res. 1167-1171 (2002) relate the expression level of the WT1 gene to a poor prognosis in breast cancer patients.

Arpino et al., Breast Cancer Res. R149-156 (2004) found that invasive lobular breast cancer was more likely to occur in older patients, larger in size, positive for estrogen receptor and for progesterone receptor, to have a lower S-phase fraction, to be negative for HER-2, p53, and EGF-receptor.

Wang et al in Anticancer Research 3637-3642 (2010) describe that the expression level of the WT1 gene might be a suitable indicator for Tamoxifen resistance in breast cancer. A differentiation between lobular and ductal breast cancer is not made, and there is no indication of a genetic marker for use as an indicator.

Phoenix et al in Annual Meeting Abstracts 419 (2010) describe SNP rs16754 as a genetic marker for acute myeloid leukemia in children.

Rakha et al in European Journal of Caner 73-83 (2008) describe that lobular breast cancer patients have a better response to hormonal therapy than other types of breast cancer. No mention is made of a genetic marker.

Damm et al in J. Clin. Oncol. 578-585 (2009) describe that the minor allele of SNP rs16754 indicates a better prognosis in acute myeloid leukemia patients. No relation to breast cancer is mentioned.

Smith et al in Eur. J. Cancer 979-982 (1987) describe the effects of endocrine therapy of lobular or ductal breast cancer in relapse patients. No mention is made of a genetic marker.

In view of the importance of adapting the therapeutic treatment to the type of breast cancer, it is the object of the invention to provide for a diagnostic marker that is predictive of the suitability of a therapeutic treatment. Preferably, the diagnostic marker is independent from known diagnostic findings.

It is a further object to provide an analytical method for use for the identification of breast cancer patients who can be treated successfully by standard therapy and for the identification of breast cancer patients who have a higher risk of relapse, e.g. 4 to 10 years after an initial cancer treatment, and who are therefore in need of additional treatment during this period. Accordingly, it is an object to provide a pharmaceutical composition for use as a medicament for treatment against the cancer in the patients that have a high risk of relapse, e.g. as a medicament for treatment within 4 to 10 years after an initial cancer treatment.

### General description of the invention

The invention attains the above-mentioned objects by the features of the claims, and especially by providing an analytical method for the determination of the susceptibility of a human patient suffering from breast cancer to anti-cancer treatment, e.g. by providing an analytical method for determining the genetic predisposition of a patient in respect of susceptibility, e.g. responsiveness, to anti-cancer treatment, especially treatment using at least one chemotherapeutical agent and/or at least one anti-hormonal agent. In greater detail, the invention provides the use of the marker Ref SNP ID rs16754 of the human WT1 gene, which is located on chromosome 11p15 (Wilms' tumour 1 gene) as an indicator in the diagnosis of nucleic acid samples originating from breast cancer patients, especially of lobular breast cancer patients, more especially in hormone receptor positive patients, as it has been found that the presence of at least one minor allele of this marker correlates with an increased risk of relapse in these patients. Further, the invention provides for the use of additional genetic markers in these embodiments, as these additional markers have been found to be coupled to the marker Ref SNP ID rs 16754 of the human WT1 gene. Accordingly, for the purposes of the invention, the reference to the marker Ref SNP ID rs 16754 (SEQ ID NO: 5) includes the reference to the group comprising or consisting of coupled markers that are given in Table 1. The sequences given for the markers including the markers of Table 1 denote the markers in their natural sequence environment as obtainable from NCBI at www.ncbi.nlm.nih.gov/snp/.

**Table 1: Markers coupled to the marker Ref SNP ID rs16754**

| marker | chromosome position | D' | r² | LOD | minor allele | major allele |
|---|---|---|---|---|---|---|
| rs5030277 (SEQ ID NO: 6) | 32374245 | 1 | 0.88 | 17.86 | A | T |
| rs5030280 (SEQ ID NO: 9) | 32374008 | 1 | 0.876 | 17.33 | C | T |
| rs5030281 (SEQ ID NO: 7) | 32373451 | 1 | 0.881 | 18.17 | T | C |
| rs5030310 (SEQ ID NO: 8) | 32367816 | 1 | 0.882 | 18.32 | T | A |
| rs11031755 (SEQ ID NO: 10) | 32351574 | 1 | 0.882 | 18.32 | T | C |
| rs11031747 (SEQ ID NO: 11) | 32343797 | 0.933 | 0.816 | 15.67 | C | T |
| rs3858439 (SEQ ID NO: 12) | 32343490 | 0.936 | 0.824 | 16.62 | C | A |
| rs3858438 (SEQ ID NO: 13) | 32343130 | 0.936 | 0.824 | 16.62 | G | C |
| rs11031746 (SEQ ID NO: 14) | 32342995 | 0.936 | 0.824 | 16.62 | C | A |
| rs11031745 (SEQ ID NO: 15) | 32342895 | 0.936 | 0.824 | 16.62 | T | C |
| rs11031744 (SEQ ID NO: 16) | 32342815 | 0.936 | 0.824 | 16.62 | T | C |
| rs3858436 (SEQ ID NO: 17) | 32342153 | 1 | 0.875 | 16.89 | G | A |
| rs2418901 (SEQ ID NO: 18) | 32341389 | 1 | 0.823 | 16.44 | G | A |

In Table 1, the chromosome position of coupled markers relates to the nucleotide position on human chromosome 11 (genome build 36.3), on which the chromosome position of marker rs16754 at nt 32374521 is a reference, in relation to which the positions of markers coupled to rs16754 are located on the WT1 gene. D' is the linkage disequilibrium (D), r is the correlation coefficient, and LOD is the LOD score estimating the recombination frequency. The alleles of the markers of table 1 that are coupled to the minor allele of marker rs16754, due to the coupling are also included in the mentioning of minor alleles and therefore are also termed minor alleles. Accordingly, for the purposes of the invention, reference to the minor allele of the marker Ref SNP ID rs16754 includes the group comprising or consisting of minor alleles of the coupled markers of table 1. Accordingly, the group comprising, preferably consisting of rs16754 and the coupled markers of table 1, especially their minor alleles form one group of markers which are indicative of lobular breast cancer patients, especially node-positive patients, who are in need of a more intense, especially a more extended treatment period, e.g. during or following 4 to 10 years after initial treatment or diagnosis, which group of markers is located at positions 32374245 to 32341389 on human chromosome 11 (genome build 36.3), wherein the members of the group of markers are closely coupled to one another, especially to marker rs16754 at position 32374521.

From Table 1, the following markers are preferred for the embodiments of the invention in addition and/or in the alternative to Ref SNP ID rs16754: rs16754, rs5030277, rs5030280, rs5030281, rs5030310, and/or rs11031755, the minor alleles of which are very closely coupled the minor allele of Ref SNP ID rs16754.

It has been found that the at least heterozygous allele G of the marker Ref SNP ID rs16754 is an indicator of a poor diagnosis in breast cancer patients, especially in lobular breast cancer patients, and therefore is an indicator identifying patients in need of intense cancer therapy and/or in need of long-term therapy, e.g. in the period of e.g. 4 to 15 years, especially 5 to 10 years after an initial cancer treatment, leading to a reduction in the proportion of relapse or progression rate of cancer. Accordingly, the analytical method of the invention allows to identify patients with the homozygous major allele of the marker Ref SNP ID rs16754 for less severe cancer treatment, because this group has a generally better prognosis, especially for long-term survival, whereas the analytical method allows to identify those patients who are in need of a more intense and/or more extended treatment, especially 4 to 10 years, preferably 5 to 10 years, more preferred 6 to 10 years after initial cancer treatment, who are the patients having at least one minor allele of the marker Ref SNP ID rs16754.

In the process for analysis, the DNA from a breast cancer patient, preferably a node-positive lobular breast cancer patient, is analysed to determine the alleles of the marker Ref SNP ID rs16754 and/or of at least one of the markers coupled to Ref SNP ID rs16754, which coupled markers are given herein. As a result of the process, the absence or presence of at least one minor allele of at least one of the markers is determined.

It is a specific advantage of the analytical method of the invention to allow the identification of these groups of patients on the basis of a parameter, namely on the basis of the presence of the minor allele of the marker Ref SNP ID rs16754, which is independent from other analytical parameters, e.g. hormone receptor status (especially independent from the status of one of ER, PR and HER2), nodal status, tumour grade, and age in multivariate analysis.

Accordingly, the invention provides an analytical method determining the alleles of the marker Ref SNP ID rs16754 in a lobular breast cancer patient, e.g. followed by the step of selecting a method of treatment in dependence on the status of alleles of the marker Ref SNP ID rs16754, which status preferably is the at least heterozygous presence of the G-allele of the marker Ref SNP ID rs16754. Further, the invention provides pharmaceutical compositions for use as a medicament, i.e. for use as an active therapeutic agent in a pharmaceutical composition, especially a hormone receptor blocking agent, most preferably for use as a medicament for the treatment of patients that are also hormone receptor positive, in the treatment of lobular breast cancer patients, which patients are characterized by the at least heterozygous allele G of the marker Ref SNP ID rs16754, e.g. as determined from a nucleic acid sample, e.g. in total DNA obtained from a cell-containing sample of the patient.

Preferably, the presence of alleles of the marker Ref SNP ID rs16754 is determined by an analytical method comprising the step of amplification of a DNA section from a DNA sample obtained from the patient, preferably a total genomic DNA sample of the patient, e.g. by PCR, optionally with the step of analysing the nucleic acid sequence of the amplificate for identifying the nucleotide present in marker Ref SNP ID rs16754, e.g. the presence of the allele G, also termed minor allele or the presence of the allele A, which is also termed major allele, e.g. by DNA sequencing. Accordingly, the invention also relates to the use of a DNA amplificate generated in vitro by amplification of a DNA section of a patient's nucleic acid, e.g. a total DNA preparation or a total nucleic acid preparation isolated from a sample of the patient, for use as an indicator for identifying a sub-group of lobular breast cancer patients, who e.g. would benefit from or require intense cancer therapy and/or long-term therapy, e.g. in the period of e.g. 4 to 15 years, especially more than 5 or 6 years to 10 years after an initial cancer treatment. Preferably, the amplificate of the patient DNA containing the marker Ref SNP ID rs16754 for use as a diagnostic indicator, e.g. as a diagnostic indicator substance identifying a lobular breast cancer patient, especially as a patient requiring intense cancer therapy and/or in need of long-term therapy, e.g. in the period of e.g. 4 to 15 years, especially 5 to 10 years after an initial cancer treatment, is an amplificate having e.g. a length of 10 to 1500 bp, and is generated by PCR using at least one pair of primers which is specific for the DNA section. Specific primer pairs for amplification of a DNA section from total patient DNA, e.g. total genomic DNA, can be designed by standard methods, and processes for identifying the allele of marker Ref SNP ID rs16754 as G and/or A are also generally known, e.g. DNA sequencing or specific hybridisation of the amplificate containing the marker, which amplificate was obtained from the genomic DNA as a template for PCR.

The marker including the reference number Ref SNP ID rs16754 is e.g. available at NCBI databank (build 129), and is also termed C1107 A>G at GenBank entry NM/024426.3 (NM-024426.3: c.1107 A>G). The nucleotide sequence of the wt1 gene according to NM024426 is enclosed as SEQ ID NO 1, wherein nucleotides 197 to 1750 contain coding sequence, with exon 7 at nucleotides 1295 to 1445, and marker Ref SNP ID rs16754 at position 1303, which corresponds to nucleotide No 1107 of the coding sequence section (nt 197 to 1750). The amino acid sequence encoded by exon 7 is given as SEQ ID NO 2, irrespective of the natural reading frame.

Preferably, the analytical method of the invention further includes at least one additional analytical step for analysis of the lobular breast cancer, which analytical step is selected from the group comprising or consisting of an analysis
in respect of its estrogen receptor status, e.g. negative or positive, as can be determined by standard analytical processes, e.g. by immuno histochemistry,
in respect of its progesteron receptor status, e.g. negative or positive as can be determined by standard analytical processes, e.g. by immuno histochemistry,
in respect of its lymph node (node) status, e.g. node negative or node positive as can be determined by standard analytical processes, e.g. by histopathology,
in respect of its HER-2 status, e.g. negative or positive as can be determined by standard analytical processes, e.g. by immuno histochemistry,
in respect of its tumour grade status, e.g. grade 2 vs. grade 1 and grade 3 vs. grade 1, e.g. can be determined by pathologic evaluation,
and in respect of patient age, e.g. younger than 50 years vs. 50 years and older.

It has been found that especially patients with lobular breast cancer who at least heterozygously have the minor allele of the marker Ref SNP ID rs 16754 who addditionally have a positive hormone receptor status, e.g. selected from estrogen receptor status, progesteron receptor status and/or HER-2 status have a significantly worse prognosis, and are therefore in need for additional therapy, e.g. in the period of 4 to 10 years following initial cancer treatment. Accordingly, these patients are the preferred patients of the invention, e.g. lobular breast cancer patients carrying at least one minor allele of the marker Ref SNP ID rs16754 and having a tumour that is positive for hormone receptor status, e.g. selected from estrogen receptor status, progesteron receptor status and/or HER-2 status, especially estrogen receptor status and progesteron receptor status.

### Detailed description of the invention

The invention is now described in greater detail with reference to the figures showing graphical representations of analytical results between breast cancer patients who are homozygous for the major allele (A/A) or have at least one minor allele (A/G and G/G) of the marker Ref SNP ID rs 16754, namely in
- Fig. 1 the OS in univariate analysis of all breast cancer patients (n=8094) analyzed,
- Fig. 2 the OS for lobular breast cancer patients only,
- Fig. 3 the breast cancer specific survival in lobular breast cancer patients,
- Fig. 4 the OS for ER positive and/or PR positive patients,
- Fig. 5 the OS for ER negative and/or PR negative patients,
- Fig. 6 the OS for lobular breast cancer patients younger than 50 years, and in
- Fig. 7 the OS for lobular breast cancer patients older than 50 years,
according to the status of Ref SNP ID rs 16754.

In the figures, year 0 of the time scale denotes the initial diagnosis and/or onset of cancer treatment.

The present invention is based on a large-scale medical study involving 8337 female breast cancer patients, in whom the alleles of the marker Ref SNP ID rs16754 were analysed in PBMC (peripheral mononuclear blood cells) and relevance of the alleles was assessed in a univariate and multivariate analyses considering age, tumour grade, lymph node involvement (nodal status), estrogen receptor status, progesterone receptor status, and HER-2 status. Generally, the data that were analysed originate from patients undergoing similar treatments, e.g. treatment was similar, irrespective of lobular or ductal breast cancer, and irrespective of the alleles of the marker Ref SNP ID rs16754.

It was found that the minor allele of the marker Ref SNP ID rs16754 is heterozygous in 25.2% of the patients and homozygous in 2.8% of the patients. Patient characteristics and pathological findings were similarly distributed between patients who are homozygous for the major allele (A/A) of the marker Ref SNP ID rs16754, and those patients who are at least heterozygous for the minor allele of the marker Ref SNP ID rs16754, i.e. heterozygous (A/G) or homozygous (G/G) for the minor allele (G).

Only in patients (n=1101) having lobular breast cancer, it was determined that the alleles of the marker Ref SNP ID rs16754 were independently predictive for overall survival (OS) in multivariate analysis (HR 1.44, 95% CI 1.06-1.94, P = 0.018), and for breast cancer specific survival with HR 1.49, 95% CI 1.07-2.07, P= 0.018, wherein HR is the hazard ratio, CI is the confidence interval, and P is the P-value indicating significance, whereas in other histological subtypes, e.g. in ductal breast cancer, a predictive value of the marker Ref SNP ID rs16754 was not found. The linkage of a minor allele of the marker Ref SNP ID rs16754 with a poor prognosis, and the need for more intense and/or longer administration of anti-cancer medicaments became more pronounced at 4 or 5 years after initial diagnosis and/or after initial treatment, and was most pronounced in hormone receptor positive and in younger lobular breast cancer patients.

Preferably, compounds for use as a medicament in the treatment of lobular breast cancer patients having at least one minor allele of the marker Ref SNP ID rs 16754 are selected from compounds used as medicaments in the treatment of hormone receptor positive breast cancer patients, especially tamoxifen, ovarian suppression agents, aromatase inhibitors, and irreversible estrogen receptor inhibitors or combinations of these, especially Tamoxifen, Anastrozol, Letrozol, and Exemestan.

### Example 1: Analysis of the marker Ref SNP ID rs16754

Analyses of the alleles of the marker Ref SNP ID rs16754 were performed using the commercially available (Applied Biosystems, Assay ID C_12034416_10) PCR-based assay specific for marker Ref SNP ID rs16754. Generally, total DNA was isolated from PBMC of breast cancer patients and subjected to PCR in microtiter plates at an annealing temperature of 60°C using the TaqMan protocol supplied by the manufacturer. For amplification, a forward primer of SEQ ID NO 3 and a backward primer of SEQ ID NO 4 were used. Detection and assessment was made with the Sequence Detection Software supplied by the manufacturer.

For statistical analyses, median follow-up time for survival was calculated according to Korn, Stat Med 5: 255-260 (1986). Overall survival (OS) endpoints were death (failure) and alive at last follow-up (generally censored at 10 years), measured from the date of entry into the study. Pair-wise comparisons of variables for exploratory purposes were done using the Kolmogorov-Smirnov test for continuous variables and the Chi-squared test for categorical variables. Estimation of distribution of OS and comparison of differences between survival curves was by the Kaplan-Meier method and the log-rank test, respectively. A Cox proportional hazards model for OS was developed for multivariate analysis and stratified by clinical study. The proportional hazards assumption was checked by scaled Schoenfeld residuals. Variables with *P* of 0.1 or below in univariate analysis were included into the model. Status of the marker Ref SNP ID rs16754 (homozygous for major allele vs. at least one minor allele), nodal status (node positive vs. node negative), estrogen receptor (ER) status (ER positive vs. ER negative), progesterone receptor (PR) status (PR positive vs. PR negative), HER-2 status (HER-2 positive vs. HER-2 negative), tumour grade (grade 2 vs. grade 1, and grade 3 vs. grade 1), and age (below 50 years vs. at least 50 years) were the variables considered.

Incomplete (more than 60%) data sets of patients were used for the multivariate Cox model following imputation of missing data using the aregImpute function of the R software package Hmisc, version 3.7, allowing the algorithm to use data from all other variables, except for the age. This procedure models the distribution of each variable present to the other variables with a predictive mean matching, and using bootstrapping to generate multiple imputations by approximating samples from the posterior predictive distributions of missing values to account for all aspects of uncertainty. Following an initial 10 data sets, a further 50 data sets were imputed. Using a logistic regression model, associations between variables and response to induction therapy were analysed. For relevance of quantitative results, odds ratios (OR) and hazard ratios (HR) were calculated at 95% CI, and the two-sided level of significance was set at P less than 0.05. For statistical analyses, the SPSS programme version 17.0 and R programme version 2.10 were used.

It was found that 71.9% of patients were homozygous for the major allele of the marker Ref SNP ID rs16754 (A/A), 25.2% were heterozygous (A/G), and 2.8% were homozygous for the minor allele of the marker Ref SNP ID rs16754 (G/G). Generally, this allele distribution corresponds to that of the general European population. When comparing pre-treatment clinical and molecular characteristics of the patients, including age, bilateral disease, tumour stage and grade, nodal status, tumour histology, ER status, PR status, ER/PR status, HER-2 status or ER/PR/HER-2 status, no significant differences were found between patients who are homozygous for the major allele to patients having at least one minor allele of the marker Ref SNP ID rs16754.

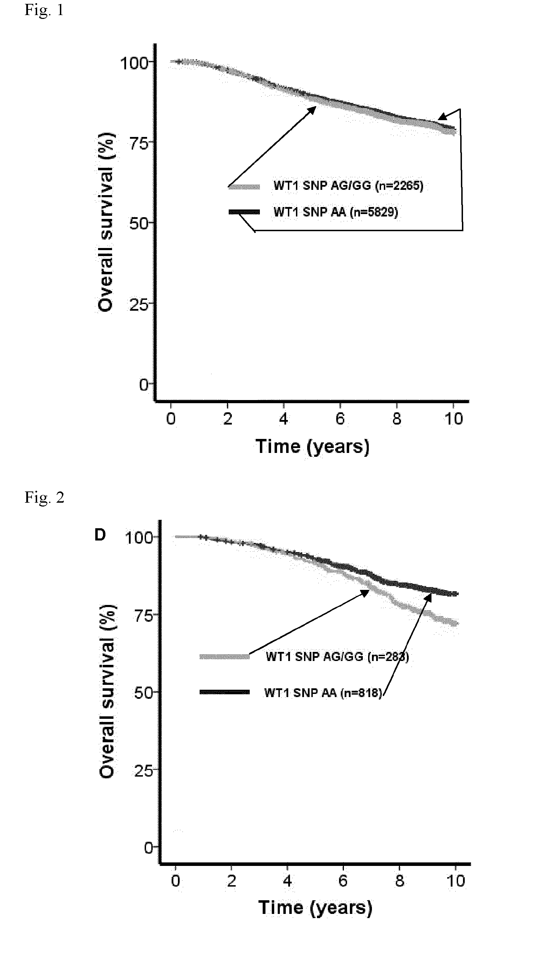

When analysing overall survival (average 9.67 years, range 0.3 to 10 years) of all breast cancer patients (n=8094; P=0.54), no significant difference was found between homozygous major alleles and at least heterozygous minor alleles of the marker Ref SNP ID rs16754, as shown in Fig. 1. In ductal carcinoma patients, OS was not significantly different between patients with at least one minor allele and patients homozygous for the major allele (lymph node negative P= 0.54, lymph node positive P=0.46, ductal carcinoma P=0.65)
Further, OS was not significantly different in node positive (n=2825) patients between patients homozygous for the major allele and patients having at least one minor allele of the marker Ref SNP ID rs16754 (P=0.41).

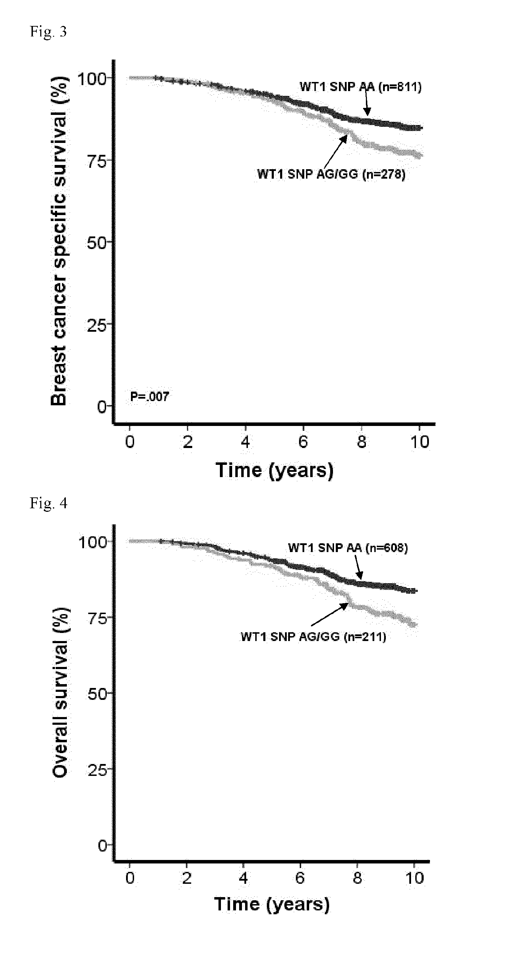

However, it was found that OS of lobular breast cancer patients having at least one minor allele of the marker Ref SNP ID rs16754 was significantly shorter (HR=1.54, 95% CI 1.15 - 2.06, P=0.004) compared to lobular breast cancer patients having the major allele of the marker Ref SNP ID rs16754 homozygously, as shown in Fig. 2. Lobular breast cancer patients having at least one minor allele of the marker Ref SNP ID rs16754 showed a 5-year OS of 88% and a 10-year OS of 71% compared to a 5-year OS of 90% and a 10-year OS of 81% for lobular breast cancer patients having the maj or allele of the marker Ref SNP ID rs16754 homozygously. It was found that the baseline characteristics were similarly distributed between patients homozygous for the major allele and patients having the minor allele of the marker Ref SNP ID rs16754, respectively, except for a higher occurrence of node positive breast cancer in patients with at least one minor allele (41% vs. 35%, P=0.058). Importantly, BCSS was significantly shorter in lobular breast cancer patients with at least one minor allele of SNP rs16754 compared to major allele homozygous patients (HR 1.55, 95% CI 1.13 - 2.14, P= 0.007) as shown in Fig. 3.

In multivariate analysis of variables having P=0.1 or below in univariate analysis, it was found that presence of at least one minor allele of the marker Ref SNP ID rs16754 in lobular breast cancer patients is associated and predictive of an increased hazard of death (HR 1.46, 95% CI 1.08 - 1.96, P=0.013). Details are shown in the following Table 2, wherein HR above 1 indicates an increased risk for the respective variable, and HR below 1 indicates a decreased risk.

**Table 2: Multivariate analysis for OS in lobular breast cancer patients (n=1089)**

| Variable | HR | 95% CI | P |
|---|---|---|---|
| at least one minor allele of the marker Ref SNP ID rs 16754 vs. homozygousity for major allele of the marker Ref SNP ID rs16754 | 1.49 | 1.07-2.07 | 0.018 |
| node positive vs. node negative | 3.24 | 2.27-4.62 | <0.001 |
| ER positive vs. ER negative | 0.82 | 0.48-1.41 | 0.47 |
| PR positive vs. PR negative | 0.58 | 0.39-0.88 | 0.01 |
| HER-2 positive vs. HER-2 negative | 1.24 | 0.54-2.85 | 0.62 |
| grade 2 vs. grade 1 | 1.42 | 0.9-2.23 | 0.13, |
| grade 3 vs. grade 1 | 2.52 | 1.31-4.83 | 0.006 |
| age ≥ 50 years vs. age < 50 years | 0.73 | 0.51-1.05 | 0.09 |

Herein, a hazard ratio (HR) above 1 indicates an increased risk for the first category listed.

### Example 2: Medicament for treatment of lobular breast cancer in patients having a minor allele of the marker Ref SNP ID rs16754

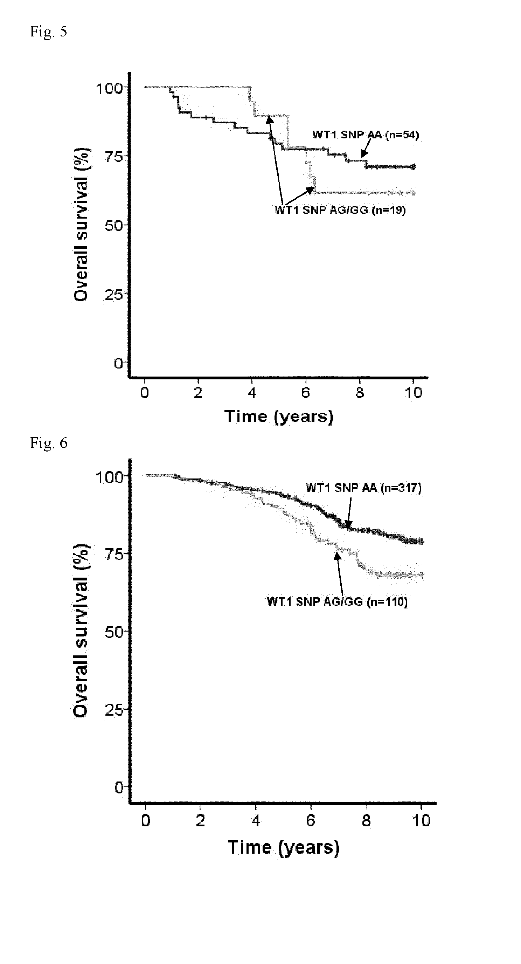

In the data analysis it became clear that the presence of at least one minor allele of the marker Ref SNP ID rs16754 in lobular breast cancer patients especially in a period of approx. 4 - 5 years after initial cancer diagnosis and treatment is correlated with a negative prognosis, i.e. a lower OS. When analysing the correlation of the alleles of the marker Ref SNP ID rs16754 to hormone receptor positive and negative patients, it was found that the presence of the minor allele was correlated with a reduced OS in hormone receptor positive patients (P=0.004, Fig. 4), but not in hormone receptor negative patients (P=0.75, Fig. 5).

In contrast to the hormone negative patients, the hormone receptor positive patients were generally treated with a hormone ablation therapy, which typically involves the administration of Tamoxifen. From the correlation of the presence of the minor allele of the marker Ref SNP ID rs16754 in lobular breast cancer patients with a reduced OS in those patients who due to the negative hormone receptor status generally did not receive treatment in the period 5 years after initial diagnosis, and the observation that presence of the minor allele is not correlated with a reduced OS in patients that receive treatment hormone ablation treatment, it can be concluded that lobular breast cancer patients having at least one minor allele of the marker Ref SNP ID rs16754 benefit from treatment with anti-hormone receptor pharmaceutical compounds irrespective of the presence of infiltrated lymph nodes.

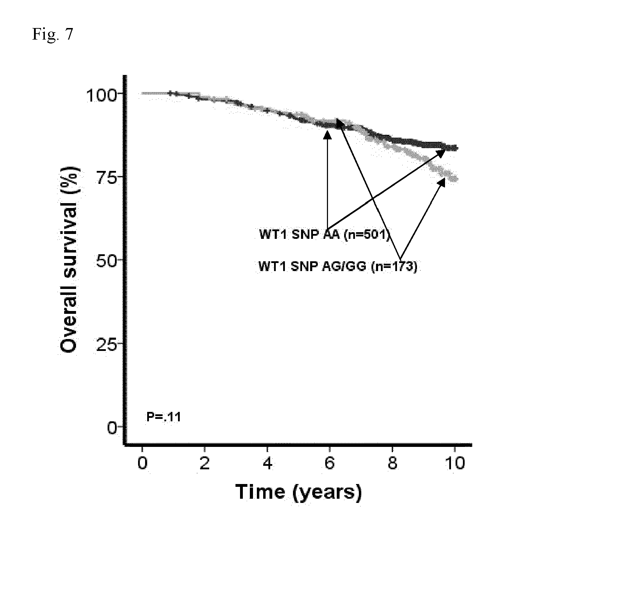

The above finding of a positive therapeutic effect of hormone ablation therapeutics in lobular breast cancer patients having at least one minor allele of the marker Ref SNP ID rs16754 was confirmed in menopausal patients. Statistical analysis showed the correlation of the presence of at least one minor allele of the marker Ref SNP ID rs16754 with reduced OS in younger patients (age of less than 50 years, mean age 43.7 years), who generally do not receive hormone ablation therapy, compared to patients who are homozygous for the major allele of the marker Ref SNP ID rs16754 (P=0.01, Fig. 6). The difference in OS was less evident in older patients (age above 50 years, mean age 59.1 years), who generally receive hormone ablation therapy, between lobular breast cancer patients having at least one minor allele of the marker Ref SNP ID rs16754 and patients who are homozygous for the major allele of the marker Ref SNP ID rs16754 (P=0.13, Fig. 7).

Hence, the invention also provides for compounds, e.g. selected from the group of pharmaceutical compounds used in hormone ablation therapy, e.g. a hormone receptor blocking agent, especially Tamoxifen, Anastrozol, Letrozol, Exemestan, for use as a medicament in the treatment of lobular breast cancer patients having at least one minor allele of the marker Ref SNP ID rs16754, especially for administration in a period of time after 4 to 5 years, e.g. up to 10 years, following initial breast cancer diagnosis or following initial breast cancer treatment.

Further, the analysis of OS in node positive lobular breast cancer patients revealed no significant difference between patients being homozygous for the major allele of the marker Ref SNP ID rs16754 and patients having at least one minor allele of the marker Ref SNP ID rs16754. When considering that generally the presence of the minor allele of the marker Ref SNP ID rs16754 is correlated with a reduced OS, the finding that node positive breast cancer patients generally receive cancer treatment by irradiation and/or chemotherapy to a higher extent than node negative patients demonstrates that especially node positive lobular breast cancer patients having at least one minor allele of the marker Ref SNP ID rs16754 benefit from the additional or harsher cancer treatment, e.g. irradiation and/or chemotherapy.
Hence, the invention also provides for compounds, e.g. selected from the group of pharmaceutical compounds used in cancer therapy, e.g. Tamoxifen, Anastrozol, Letrozol or Exemestan for use as a medicament in the treatment of lobular breast cancer patients having at least one minor allele of the marker Ref SNP ID rs16754 or having the coupled allele of a marker coupled to the marker Ref SNP ID rs16754, especially for administration in a period of time after 4 to 5 years following initial breast cancer diagnosis or treatment, especially those patients who are node positive and/or patients who are node negative.

## Claims

1. Process for the analysis of the susceptibility of a human patient to anti-cancer treatment, **characterized by** analysing the alleles of the marker Ref SNP ID rs16754 and/or of the alleles of at least one marker of the group of markers comprising rs5030277, rs5030280, rs5030281, rs5030310, rs11031755, rs11031747, rs3858439, rs3858438, rs11031746, rs11031745, rs11031744, rs3858436, rs2418901, in a nucleic acid sample obtained from a human suffering from lobular breast cancer.

2. Process according to claim 1, **characterized in that** the breast cancer has at least one positive hormone receptor status.

3. Process according to claim 2, **characterized in that** the positive hormone receptor status is at least one of estrogen receptor status, progesteron receptor status and/or HER-2 status.

4. Process according to one of the preceding claims, **characterized in that** the process is for use in the selection of a therapy against lobular breast cancer.

5. Process according to one of the preceding claims, **characterized in that** the nucleic acid sample is provided, and the alleles of the marker Ref SNP ID rs16754 and/or the alleles of at least one of the markers of the group comprising rs5030277, rs5030280, rs5030281, rs5030310, rs11031755, rs11031747, rs3858439, rs3858438, rs11031746, rs11031745, rs11031744, rs3858436, rs2418901 are determined, and the hormone receptor status of the patient is determined.

6. Process according to one of the preceding claims, **characterized in that** the hormone receptor status of the cancer is determined.

7. Use of a kit of parts containing oligonucleotides which are complementary to at least a section of the human WT1 gene corresponding to SEQ ID NO 1, the section comprising at least one of the markers selected from the group consisting of SNP ID rs16754, rs5030277, rs5030280, rs5030281, rs5030310, rs11031755, rs11031747, rs3858439, rs3858438, rs11031746, rs11031745, rs11031744, rs3858436, and rs2418901 as reagents for the analysis of the allele of the marker Ref SNP ID rs16754 and/or of the allele of at least one of the markers of the group comprising rs5030277, rs5030280, rs5030281, rs5030310, rs11031755, rs11031747, rs3858439, rs3858438, rs11031746, rs11031745, rs11031744, rs3858436, rs2418901 in a process according to one of the preceding claims in a nucleic acid sample obtained from a human patient suffering from lobular breast cancer.

8. Pharmaceutical composition containing Tamoxifen, Anastrozol, Letrozol or Exemestan as an active compound for use as a medicament in the treatment of lobular breast cancer in patients who are **characterized by** bearing the allele G in marker Ref SNP ID rs16754 at least heterozygously, or the minor allele of at least one of the markers of the group comprising rs5030277, rs5030280, rs5030281, rs5030310, rs11031755, rs11031747, rs3858439, rs3858438, rs11031746, rs11031745, rs11031744, rs3858436, rs2418901 at least heterozygously.

9. Pharmaceutical composition according to claim 8, **characterized in that** the medicament is for use in the treatment for at least 5 years to 10 years, preferably 6 years to 10 years, following the detection of breast cancer.

10. Pharmaceutical composition according to claim 8 or 9, **characterized in that** the patients have received treatment against breast cancer 5 to 10 years previously.

11. Pharmaceutical composition according to one of claims 8 to 10, **characterized by** containing a hormone receptor blocking agent as the pharmaceutically active compound.

12. Process for analysis of the alleles of the marker Ref SNP ID rs16754 or of the alleles of at least one of the markers of the group comprising rs5030277, rs5030280, rs5030281, rs5030310, rs11031755, rs11031747, rs3858439, rs3858438, rs11031746, rs11031745, rs11031744, rs3858436, rs2418901 in a nucleic acid sample, **characterized in that** the nucleic acid sample was obtained from a human patient suffering from lobular breast cancer, for use in the selection of a therapy in a period of 3 to 10 years following the initial detection of breast cancer, wherein the breast cancer has at least one positive hormone receptor status selected from the group of estrogen receptor status, progesteron receptor status and/or HER-2 status.

13. Use of an amplified section of the human WT1 gene as an indicator substance for identifying a sub-group from lobular breast cancer patients, which section is obtainable by enzymatic DNA amplification from a DNA sample originating from a lobular breast cancer patient, which section comprises the marker Ref SNP ID rs16754 and/or at least one of the markers from the group comprising rs5030277, rs5030280, rs5030281, rs5030310, rs11031755, rs11031747, rs3858439, rs3858438, rs11031746, rs11031745, rs11031744, rs3858436, rs2418901.

14. Use according to claim 13, wherein the patients are identified by the presence of at least one G-allele of the marker Ref SNP ID rs16754 and/or at least one minor allele of at least one of the markers of the group consisting of rs5030277, rs5030280, rs5030281, rs5030310, rs11031755, rs11031747, rs3858439, rs3858438, rs11031746, rs11031745, rs11031744, rs3858436, and rs2418901.

15. Use according to one of claims 13 to 14, **characterized in that** the amplified DNA section comprises nucleotides 32374245 to 32341389 of human chromosome 11.


SEQUENCE LISTING

<110> Medizinische Hochschule Hannover

<120> Prognostic marker for mamma carcinoma

<130> M1031-N-EP

<150> EPEP10193883
<151> 2010-12-06
<150> EPEP10194061
<151> 2010-12-07

<160> 18

<170> BiSSAP 1.0

<210> 1
<211> 3029
<212> DNA
<213> Homo sapiens

<220>
<221> source
<222> 1..3029
<223> /mol_type="DNA"
      /organism="Homo sapiens"

<220>
<221> CDS
<222> 197..1750
<223> /codon_start=1
      /gene="exon 7"
      /note="SNP ID rs16754"
      /transl_table=1
      /translation="LQDPASTCVPEPASQHTLRSGPGCLQQPEQQGVRDPGGIWAKLGAAEASAE
      RLQGRRSRGASGSEPQQMGSDVRDLNALLPAVPSLGGGGGCALPVSGAAQWAPVLDFAPPGASAY
      GSLGGPAPPPAPPPPPPPPPHSFIKQEPSWGGAEPHEEQCLSAFTVHFSGQFTGTAGACRYGPFG
      PPPPSQASSGQARMFPNAPYLPSCLESQPAIRNQGYSTVTFDGTPSYGHTPSHHAAQFPNHSFKH
      EDPMGQQGSLGEQQYSVPPPVYGCHTPTDSCTGSQALLLRTPYSSDNLYQMTSQLECMTWNQMNL
      GATLKGVAAGSSSSVKWTEGQSNHSTGYESDNHTTPILCGAQYRIHTHGVFRGIQDVRRVPGVAP
      TLVRSASETSEKRPFMCAYPGCNKRYFKLSHLQMHSRKHTGEKPYQCDFKDCERRFSRSDQLKRH
      QRRHTGVKPFQCKTCQRKFSRSDHLKTHTRTHTGKTSEKPFSCRWPSCQKKFARSDELVRHHNMH
      QRNMTKLQLAL"

<400> 1
   ccaggcagct ggggtaagga gttcaaggca gcgcccacac ccgggggctc tccgcaaccc      60
   gaccgcctgt ccgctccccc acttcccgcc ctccctccca cctactcatt cacccaccca     120
   cccacccaga gccgggacgg cagcccaggc gcccgggccc cgccgtctcc tcgccgcgat     180
   cctggacttc ctcttgctgc aggacccggc ttccacgtgt gtcccggagc cggcgtctca     240
   gcacacgctc cgctccgggc ctgggtgcct acagcagcca gagcagcagg gagtccggga     300
   cccgggcggc atctggccca agttaggcgc cgccgaggcc agcgctgaac gtctccaggg     360
   ccggaggagc cgcggggcgt ccgggtctga gccgcagcaa atgggctccg acgtgcggga     420
   cctgaacgcg ctgctgcccg ccgtcccctc cctgggtggc ggcggcggct gtgccctgcc     480
   tgtgagcggc gcggcgcagt gggcgccggt gctggacttt gcgcccccgg gcgcttcggc     540
   ttacgggtcg ttgggcggcc ccgcgccgcc accggctccg ccgccacccc cgccgccgcc     600
   gcctcactcc ttcatcaaac aggagccgag ctggggcggc gcggagccgc acgaggagca 660
   gtgcctgagc gccttcactg tccacttttc cggccagttc actggcacag ccggagcctg 720
   tcgctacggg cccttcggtc ctcctccgcc cagccaggcg tcatccggcc aggccaggat 780
   gtttcctaac gcgccctacc tgcccagctg cctcgagagc cagcccgcta ttcgcaatca 840
   gggttacagc acggtcacct tcgacgggac gcccagctac ggtcacacgc cctcgcacca 900
   tgcggcgcag ttccccaacc actcattcaa gcatgaggat cccatgggcc agcagggctc 960
   gctgggtgag cagcagtact cggtgccgcc cccggtctat ggctgccaca cccccaccga 1020
   cagctgcacc ggcagccagg ctttgctgct gaggacgccc tacagcagtg acaatttata 1080
   ccaaatgaca tcccagcttg aatgcatgac ctggaatcag atgaacttag gagccacctt 1140
   aaagggagtt gctgctggga gctccagctc agtgaaatgg acagaagggc agagcaacca 1200
   cagcacaggg tacgagagcg ataaccacac aacgcccatc ctctgcggag cccaatacag 1260
   aatacacacg cacggtgtct tcagaggcat tcaggatgtg cgacgtgtgc ctggagtagc 1320
   cccgactctt gtacggtcgg catctgagac cagtgagaaa cgccccttca tgtgtgctta 1380
   cccaggctgc aataagagat attttaagct gtcccactta cagatgcaca gcaggaagca 1440
   cactggtgag aaaccatacc agtgtgactt caaggactgt gaacgaaggt tttctcgttc 1500
   agaccagctc aaaagacacc aaaggagaca tacaggtgtg aaaccattcc agtgtaaaac 1560
   ttgtcagcga aagttctccc ggtccgacca cctgaagacc cacaccagga ctcatacagg 1620
   taaaacaagt gaaaagccct tcagctgtcg gtggccaagt tgtcagaaaa agtttgcccg 1680
   gtcagatgaa ttagtccgcc atcacaacat gcatcagaga aacatgacca aactccagct 1740
   ggcgctttga ggggtctccc tcggggaccg ttcagtgtcc caggcagcac agtgtgtgaa 1800
   ctgctttcaa gtctgactct ccactcctcc tcactaaaaa ggaaacttca gttgatcttc 1860
   ttcatccaac ttccaagaca agataccggt gcttctggaa actaccaggt gtgcctggaa 1920
   gagttggtct ctgccctgcc tacttttagt tgactcacag gccctggaga agcagctaac 1980
   aatgtctggt tagttaaaag cccattgcca tttggtgtgg attttctact gtaagaagag 2040
   ccatagctga tcatgtcccc ctgacccttc ccttcttttt ttatgctcgt tttcgctggg 2100
   gatggaatta ttgtaccatt ttctatcatg gaatatttat aggccagggc atgtgtatgt 2160
   gtctgctaat gtaaactttg tcatggtttc catttactaa cagcaacagc aagaaataaa 2220
   tcagagagca aggcatcggg ggtgaatctt gtctaacatt cccgaggtca gccaggctgc 2280
   taacctggaa agcaggatgt agttctgcca ggcaactttt aaagctcatg catttcaagc 2340
   agctgaagaa aaaatcagaa ctaaccagta cctctgtata gaaatctaaa agaattttac 2400
   cattcagtta attcaatgtg aacactggca cactgctctt aagaaactat gaagatctga 2460
   gatttttttg tgtatgtttt tgactctttt gagtggtaat catatgtgtc tttatagatg 2520
   tacatacctc cttgcacaaa tggaggggaa ttcattttca tcactgggag tgtccttagt 2580
   gtataaaaac catgctggta tatggcttca agttgtaaaa atgaaagtga ctttaaaaga 2640
   aaatagggga tggtccagga tctccactga taagactgtt tttaagtaac ttaaggacct 2700
   ttgggtctac aagtatatgt gaaaaaaatg agacttactg ggtgaggaaa tccattgttt 2760
   aaagatggtc gtgtgtgtgt gtgtgtgtgt gtgtgtgtgt gtgttgtgtt gtgttttgtt 2820
   ttttaaggga gggaatttat tatttaccgt tgcttgaaat tactgtgtaa atatatgtct 2880
   gataatgatt tgctctttga caactaaaat taggactgta taagtactag atgcatcact 2940
   gggtgttgat cttacaagat attgatgata acacttaaaa ttgtaacctg catttttcac 3000
   tttgctctca attaaagtct attcaaaag 3029

<210> 2
<211> 517
<212> PRT
<213> Homo sapiens

<220>
<221> SOURCE
<222> 1..517
<223> /mol_type="protein"
/note="This sequence is generated from feature [CDS]:197..1750 pl
aced under sequence number 1"
/organism="Homo sapiens"

<400> 2
Leu Gln Asp Pro Ala Ser Thr Cys Val Pro Glu Pro Ala Ser Gln His
1               5                   10                  15
Thr Leu Arg Ser Gly Pro Gly Cys Leu Gln Gln Pro Glu Gln Gln Gly
            20                  25                  30
Val Arg Asp Pro Gly Gly Ile Trp Ala Lys Leu Gly Ala Ala Glu Ala
        35                  40                  45
Ser Ala Glu Arg Leu Gln Gly Arg Arg Ser Arg Gly Ala Ser Gly Ser
    50                  55                  60
Glu Pro Gln Gln Met Gly Ser Asp Val Arg Asp Leu Asn Ala Leu Leu
65                  70                  75                  80
Pro Ala Val Pro Ser Leu Gly Gly Gly Gly Gly Cys Ala Leu Pro Val
                85                  90                  95
Ser Gly Ala Ala Gln Trp Ala Pro Val Leu Asp Phe Ala Pro Pro Gly
            100                 105                 110
Ala Ser Ala Tyr Gly Ser Leu Gly Gly Pro Ala Pro Pro Pro Ala Pro
        115                 120                 125
Pro Pro Pro Pro Pro Pro Pro Pro His Ser Phe Ile Lys Gln Glu Pro
    130                 135                 140
Ser Trp Gly Gly Ala Glu Pro His Glu Glu Gln Cys Leu Ser Ala Phe
145                 150                 155                 160
Thr Val His Phe Ser Gly Gln Phe Thr Gly Thr Ala Gly Ala Cys Arg
                165                 170                 175
Tyr Gly Pro Phe Gly Pro Pro Pro Pro Ser Gln Ala Ser Ser Gly Gln
            180                 185                 190
Ala Arg Met Phe Pro Asn Ala Pro Tyr Leu Pro Ser Cys Leu Glu Ser
        195                 200                 205
Gln Pro Ala Ile Arg Asn Gln Gly Tyr Ser Thr Val Thr Phe Asp Gly
    210                 215                 220
Thr Pro Ser Tyr Gly His Thr Pro Ser His His Ala Ala Gln Phe Pro
225                 230                 235                 240
Asn His Ser Phe Lys His Glu Asp Pro Met Gly Gln Gln Gly Ser Leu
                245                 250                 255

Gly Glu Gln Gln Tyr Ser Val Pro Pro Pro Val Tyr Gly Cys His Thr
            260                 265                 270
Pro Thr Asp Ser Cys Thr Gly Ser Gln Ala Leu Leu Leu Arg Thr Pro
        275                 280                 285
Tyr Ser Ser Asp Asn Leu Tyr Gln Met Thr Ser Gln Leu Glu Cys Met
    290                 295                 300
Thr Trp Asn Gln Met Asn Leu Gly Ala Thr Leu Lys Gly Val Ala Ala
305                 310                 315                 320
Gly Ser Ser Ser Ser Val Lys Trp Thr Glu Gly Gln Ser Asn His Ser
                325                 330                 335
Thr Gly Tyr Glu Ser Asp Asn His Thr Thr Pro Ile Leu Cys Gly Ala
            340                 345                 350
Gln Tyr Arg Ile His Thr His Gly Val Phe Arg Gly Ile Gln Asp Val
        355                 360                 365
Arg Arg Val Pro Gly Val Ala Pro Thr Leu Val Arg Ser Ala Ser Glu
    370                 375                 380
Thr Ser Glu Lys Arg Pro Phe Met Cys Ala Tyr Pro Gly Cys Asn Lys
385                 390                 395                 400
Arg Tyr Phe Lys Leu Ser His Leu Gln Met His Ser Arg Lys His Thr
                405                 410                 415
Gly Glu Lys Pro Tyr Gln Cys Asp Phe Lys Asp Cys Glu Arg Arg Phe
            420                 425                 430
Ser Arg Ser Asp Gln Leu Lys Arg His Gln Arg Arg His Thr Gly Val
        435                 440                 445
Lys Pro Phe Gln Cys Lys Thr Cys Gln Arg Lys Phe Ser Arg Ser Asp
    450                 455                 460
His Leu Lys Thr His Thr Arg Thr His Thr Gly Lys Thr Ser Glu Lys
465                 470                 475                 480
Pro Phe Ser Cys Arg Trp Pro Ser Cys Gln Lys Lys Phe Ala Arg Ser
                485                 490                 495
Asp Glu Leu Val Arg His His Asn Met His Gln Arg Asn Met Thr Lys
            500                 505                 510
Leu Gln Leu Ala Leu
        515

<210> 3
<211> 21
<212> DNA
<213> artificial sequences

<220>
<221> source
<222> 1..21
<223> /PCR_primers="[fwd_name: SEQ ID NO 3]"
      /mol_type="DNA"
      /note="SEQ ID NO 3, forward primer"
      /organism="artificial sequences"

<400> 3
ctccagtgct cactctccct c                                              21


<210> 4
<211> 21
<212> DNA
<213> artificial sequences

<220>
<221> source
<222> 1..21
<223> /PCR_primers="[rev_name: SEQ ID NO 4]"
      /mol_type="DNA"
      /note="SEQ ID NO 4, backward primer"
      /organism="artificial sequences"

<400> 4
ccttagcagt gtgagagcct g                                                21


<210> 5
<211> 511
<212> DNA
<213> Homo sapiens

<220>
<221> source
<222> 1..511
<223> /mol_type="DNA"
      /note="rs16754"
      /organism="Homo sapiens"

<220>
<221> misc_difference
<222> 256
<223> /allele="alleles='A/G'"

<400> 5
ttcatctcaa cagccacttc tggttgctca gtagtgatga tcttagagca ctgcattgta      60

gaatgtcctg atctagcatc cattactgat gtctgccatg gggatctgga gtgtgaatgg     120

gagtggtttt aggtctgcac ctgccacccc ttctttggat atactccagt gctcactctc     180

cctcaagacc tacgtgaatg ttcacatgtg gcttaaagcc tcccttcctc ttactctctg     240

cctgcaggat gtgcgrcgtg tgcctggagt agccccgact cttgtacggt cggcatctga     300

gaccagtgag aaacgcccct tcatgtgtgc ttacccaggc tgcaataaga gatattttaa     360

gctgtcccac ttacagatgc acagcaggaa gcacactggt aagtgtgccc gctgtccagt     420

cttgggcaaa catggttcaa gagctccttt tccaggctct cacactgcta aggacccagg     480

ttatttccag gatggaaagt aagcactgct t                                    511


<210> 6
<211> 511
<212> DNA
<213> Homo sapiens

<220>
<221> source
<222> 1..511
<223> /mol_type="DNA"
      /note="rs5030277"
      /organism="Homo sapiens"

<220>
<221> misc_difference
<222> 256
<223> /allele="alleles='A/T'"

<400> 6
gactcttgta cggtcggcat ctgagaccag tgagaaacgc cccttcatgt gtgcttaccc      60

aggctgcaat aagagatatt ttaagctgtc ccacttacag atgcacagca ggaagcacac     120

tggtaagtgt gcccgctgtc cagtcttggg caaacatggt tcaagagctc cttttccagg     180

ctctcacact gctaaggacc caggttattt ccaggatgga aagtaagcac tgctttgaaa 240

ggtctgatcc aggtgwtgta aagaggctgt ggggccattt tgcattctga ttatctatcc 300

tttattacag aggtcagtaa actgtcttcc agaaaggcag agcagagtgg tagagagaaa 360

gtgccacaga attaaagaga cctgtgtagg aatcgtgttg gtgttagaat actattagca 420

tttctaagga gagaggagct ggcctgggaa tatagtcatc agatggtttt aggaggtcga 480

tgggcaagat cattgtgaaa ttttagaaga g 511

<210> 7
<211> 511
<212> DNA
<213> Homo sapiens

<220>
<221> source
<222> 1..511
<223> /mol_type="DNA"
/note="rs5030281"
/organism="Homo sapiens"

<220>
<221> misc_difference
<222> 256
<223> /allele="alleles='C/T'"

<400> 7
caggagaatt gcttgaacct aggaggcaga ggttgcagtg agctgagatc ttaccactgt 60

actccagcct aggcgacaga gcaagactct gccaaaaaaa aaaaaaagga gagagagaga 120

tgatgatttt ttatctttga agatacgcca atgaaactgg attttccccc aggtcagaga 180

ggctgcttct gggaaacaga gaactggcat tgtttggtgt tggccacctt gaatgtggac 240

agccatgtca cagaaygttt actctgggcc ttgtgggcac taggtgggag tgtgggagtg 300

tggtaaaacc caggttttcc tacagaggag ctactgctgc cttttccaca atcgaagaac 360

agcattatta taggatcact gttgcaactg tgttattact gctgattttg ctcttgggaa 420

gctggagaaa aaagtgagct gcattcagat ttccttggac cattgaaatt tgctaattca 480

ctgtgtccag aggatgcaat tctggtgtca a 511

<210> 8
<211> 511
<212> DNA
<213> Homo sapiens

<220>
<221> source
<222> 1..511
<223> /mol_type="DNA"
/note="rs5030310"
/organism="Homo sapiens"

<220>
<221> misc_difference
<222> 256

<223> /allele="alleles='A/T'"

<400> 8
ccagcacaag gcttggcata tgttaagcat caaataaatg gtagttattc tccctaatga 60

gaatgaggct tggaaagaat gtctgagggc tctttgggtg gagaagcctc aatcttttgg 120

caagcttatt aaaaatctaa gaaccacgcc ccaactagaa tcttccatat aacaggatta 180

tgctctcagt aacactttaa gagaaaaaaa aaaacaacac ataacatgta aactggtatc 240

agtgatttat tttaawgtat ccagcccatc cagggtatct acacagatag aagtgttaaa 300

agcttcagtc acagctgaga tccctggaca gtgatcactc agtaataccc agggtagatg 360

acccagaggc ttctgcagcc cagttcatct cagttcccaa cttactcaac aagggggaga 420

aatttacaca gttcacaaaa cattctatca gcagagactg aaacaaacgt ttttcccctt 480

aattatgaat tgctcagaca tttcccccaa c 511


<210> 9
<211> 685
<212> DNA
<213> Homo sapiens

<220>
<221> source
<222> 1..685
<223> /mol_type="DNA"
      /note="|rs5030280"
      /organism="Homo sapiens"

<220>
<221> misc_difference
<222> 201
<223> /allele="alleles='C/T'"

<400> 9
atctatcctt tattacagag gtcagtaaac tgtcttccag aaaggcagag cagagtggta 60

gagagaaagt gccacagaat taaagagacc tgtgtaggaa tcgtgttggt gttagaatac 120

tattagcatt tctaaggaga gaggagctgg cctgggaata tagtcatcag atggttttag 180

gaggtcgatg ggcaagatca ytgtgaaatt ttagaagaga tggtccaaag aaaaacttac 240

ctgcttgggt cagtaataat caactagaca ggaaggggaa aattgattgt tagtttgatt 300

ttttagtgat cacttaaaaa aaaaaggtca gtcatggggg ctcacgcctg taatcccagc 360

actttgggag gccgaagtgg gtggatcacc caaggtcagg agttggagac cagcctggcc 420

aacaaggtga aaccccgtct ctactaaaaa tacaaaaatg agccgggtgt ggtggcacgc 480

acctgtactt gggaggctgg ggcaggagaa ttgcttgaac ctaggaggca gaggttgcag 540

tgagctgaga tcttaccact gtactccagc ctgggcgaca gagcaagact ctgccaaaaa 600

aaaaaaaaag gagagagaga gatgatgatt ttttatcttt gaagatacgc caatgaaact 660

ggattttccc ccaggtcaga gaggc 685

<210> 10
<211> 773
<212> DNA
<213> Homo sapiens

<220>
<221> source
<222> 1..773
<223> /mol_type="DNA"
      /note="rs11031755"
      /organism="Homo sapiens"

<220>
<221> misc_difference
<222> 323
<223> /allele="alleles='C/T'"

<400> 10
caaattcaat ttatacaatc atctcagtta acaggccatt ccttttcccg atattttgag      60

agaattcatg taataaaggg attatttata gtgccgtcga aagagcattg acttgggagg     120

aaaacagaac tgctcttgat gtgagttggc cactcaccag ccttatgacc tagcaaatta     180

tttcatctct ctttcagtat cttccacatc actgagcaag tggatggaga gaggaaagac     240

acctggtgaa tcccggtgat aagactgatg attggaaata aaatttgcca tgaaatggga     300

caagcatact ccagtatgac tgyatcacaa ttaattgcat ctgcaatgac cttattccca     360

aataaagtca cattctgagg tgacgtgaat tttggaatga cactatccag accacacacc     420

tatattcatt catatttgga aactccattt cgggttatga aacacgtgcc tcacttgttg     480

catgcccttt gcagacaggg tgatgcaaag tgtgacacag tgtgacctgg gttccagtca     540

ttgtcctgcc ttgtgctaga tgaccctggg caatctaatt catctttcta ggcctcatcc     600

aaataatgaa gtcaggagag gtggtctcta agaacccttg gaatttaatt attcacaagc     660

ttttgggact caattaaaca tttttttctt ttcttttttt tttttttttt tttttttttt     720

tcaggggaaa tgtaggaatg agccatcatg aaacgatgcc agaggctgga gag           773


<210> 11
<211> 1448
<212> DNA
<213> Homo sapiens

<220>
<221> source
<222> 1..1448
<223> /mol_type="DNA"
      /note="rs11031747"
      /organism="Homo sapiens"

<220>
<221> misc_difference
<222> 745
<223> /allele="|alleles='C/T'"

<400> 11
catcagttag catgaaccaa atacagatgg ggaaaataac taccctgagt ctgctcactc      60

ctgcaattct aggatcagac atcccaggtg aggagtcccc atgaagcggc atcgtttgtc 120

tggggtaata cctgaggttg gttgcctcat gccaaggaaa tcaaggacac ggacacacgt 180

ggagtgaaga ttaagaatgg aggtttaata ggcgaaatta agaaaaaaga gaaatagctc 240

tcccttctgc agagagagag gagatcccga gggggtcttc tggtttggtg gtgaaatgca 300

cagggtttta tagtctggtt tgcggaggct gtgtctgact tacatagggc ccaaagattg 360

gttggaccag gcatgacatt tatacagccc gtgaagaagt tggccacccc accctaatct 420

tttgttatgc aaatgagttt ttttaactgg ccagcaccac attgtcagct ccttactgta 480

cacatggttg ataaaggaaa gggaagatgg agcctccatg ttggacatgt ctagccccca 540

ggtagccttt tcctattgtc acagctgcag gcattcacct ctgcaagctt ccgacttgct 600

tatctatgtt tgcagctcaa ttttacaggc tgctttttgt tagaaaagaa atgatttggc 660

cgggcgcagt ggctcatgcc tgtaatccca gcactttggg aggccgaggc aggcggatca 720

cctgaggtca ggagttcaag gcccygtctc tctactaaaa atacaaaaat tagccgggcg 780

tgggggccgg cacctgtaat tccagctact tggaggctga ggcaggagaa tcgcttgaat 840

ccggaaggcg gaggttgcag tgagccgaga tggcgccact gcacttcagt ctaggcaaca 900

agagcgaaac tccgtctcaa aaaaaaaaaa aaaaaaacac tggggggctg ctctttatga 960

aaagggaaac cttaccaagg acttccttat cctcactatc tgtctaaata atttcttctt 1020

aactcctgta tcacccagat atctccgtga ggagtcagga gggaaatttg ggctgattgc 1080

tgggtgtttt tgtttttgtt tgtttttggt tttttttgag acggagtctc gggcagctac 1140

ccaggctgga gtgcagtggt gcaatctcgg ctcactgcaa gctccgcctc ctgggttcac 1200

gccattcttc tgcctcagcc tccggagtag cagggactac aggcatccgc caccacgcct 1260

ggctaatttt ttgtgttttt agtagagacg gggtttcacc gtgttagcca agatggtctc 1320

tatctactga cctcgtgatc cgcccccctc agcctcccaa agtgctggaa ttacaggcat 1380

gagccaccga ccccggccca ggctggtttg tttttaatga gaggctgggt ggctgttagg 1440

gaggggga 1448

<210> 12
<211> 1448
<212> DNA
<213> Homo sapiens

<220>
<221> source
<222> 1..1448
<223> /mol_type="DNA"
/note="rs3858439"
/organism="Homo sapiens"

<220>
<221> misc_difference
<222> 1011
<223> /allele="alleles='A/C'"

<400> 12
tccccctccc taacagccac ccagcctctc attaaaaaca aaccagcctg ggccggggtc      60

ggtggctcat gcctgtaatt ccagcacttt gggaggctga ggggggcgga tcacgaggtc      120

agtagataga gaccatcttg gctaacacgg tgaaaccccg tctctactaa aaacacaaaa      180

aattagccag gcgtggtggc ggatgcctgt agtccctgct actccggagg ctgaggcaga      240

agaatggcgt gaacccagga ggcggagctt gcagtgagcc gagattgcac cactgcactc      300

cagcctgggt agctgcccga gactccgtct caaaaaaaac caaaaacaaa caaaaacaaa      360

aacacccagc aatcagccca aatttccctc ctgactcctc acggagatat ctgggtgata      420

caggagttaa gaagaaatta tttagacaga tagtgaggat aaggaagtcc ttggtaaggt      480

ttcccttttc ataaagagca gccccccagt gttttttttt tttttttttt gagacggagt      540

ttcgctcttg ttgcctagac tgaagtgcag tggcgccatc tcggctcact gcaacctccg      600

ccttccggat tcaagcgatt ctcctgcctc agcctccaag tagctggaat tacaggtgcc      660

ggcccccacg cccggctaat ttttgtattt ttagtagaga gacagggcct tgaactcctg      720

acctcaggtg atccgcctgc ctcggcctcc caaagtgctg ggattacagg catgagccac      780

tgcgcccggc caaatcattt cttttctaac aaaaagcagc ctgtaaaatt gagctgcaaa      840

catagataag caagtcggaa gcttgcagag gtgaatgcct gcagctgtga caataggaaa      900

aggctacctg ggggctagac atgtccaaca tggaggctcc atcttccctt tcctttatca      960

accatgtgta cagtaaggag ctgacaatgt ggtgctggcc agttaaaaaa mctcatttgc      1020

ataacaaaag attagggtgg ggtggccaac ttcttcacgg gctgtataaa tgtcatgcct      1080

ggtccaacca atctttgggc cctatgtaag tcagacacag cctccgcaaa ccagactata      1140

aaaccctgtg catttcacca ccaaaccaga agaccccctc gggatctcct ctctctctgc      1200

agaaggaga gctatttctc tttttcttta atttcgccta ttaaacctcc attcttaatc      1260

ttcactccac gtgtgtccgt gtccttgatt tccttggcat gaggcaacca acctcaggta      1320

ttaccccaga caaacgatgc cgcttcatgg ggactcctca cctgggatgt ctgatcctag      1380

aattgcagga gtgagcagac tcagggtagt tattttcccc atctgtattt ggttcatgct      1440

aactgatg                                                              1448

<210> 13
<211> 1548
<212> DNA
<213> Homo sapiens

<220>
<221> source
<222> 1..1548
<223> /mol_type="DNA"
      /note="rs3858438"
      /organism="Homo sapiens"

<220>
<221> misc_difference
<222> 1348
<223> /allele="alleles='C/G'"

<400> 13
gcctctcatt aaaaacaaac cagcctgggc cggggtcggt ggctcatgcc tgtaattcca 60

gcactttggg aggctgaggg gggcggatca cgaggtcagt agatagagac catcttggct 120

aacacggtga aaccccgtct ctactaaaaa cacaaaaaat tagccaggcg tggtggcgga 180

tgcctgtagt ccctgctact ccggaggctg aggcagaaga atggcgtgaa cccaggaggc 240

ggagcttgca gtgagccgag attgcaccac tgcactccag cctgggtagc tgcccgagac 300

tccgtctcaa aaaaaaccaa aaacaaacaa aaacaaaaac acccagcaat cagcccaaat 360

ttccctcctg actcctcacg gagatatctg ggtgatacag gagttaagaa gaaattattt 420

agacagatag tgaggataag gaagtccttg gtaaggtttc ccttttcata aagagcagcc 480

ccccagtgtt tttttttttt tttttttgag acggagtttc gctcttgttg cctagactga 540

agtgcagtgg cgccatctcg gctcactgca acctccgcct tccggattca agcgattctc 600

ctgcctcagc ctccaagtag ctggaattac aggtgccggc ccccacgccc ggctaatttt 660

tgtattttta gtagagagac agggccttga actcctgacc tcaggtgatc cgcctgcctc 720

ggcctcccaa agtgctggga ttacaggcat gagccactgc gcccggccaa atcatttctt 780

ttctaacaaa aagcagcctg taaaattgag ctgcaaacat agataagcaa gtcggaagct 840

tgcagaggtg aatgcctgca gctgtgacaa taggaaaagg ctacctgggg gctagacatg 900

tccaacatgg aggctccatc ttccctttcc tttatcaacc atgtgtacag taaggagctg 960

acaatgtggt gctggccagt taaaaaaact catttgcata acaaaagatt agggtggggt 1020

ggccaacttc ttcacgggct gtataaatgt catgcctggt ccaaccaatc tttgggccct 1080

atgtaagtca gacacagcct ccgcaaacca gactataaaa ccctgtgcat ttcaccacca 1140

aaccagaaga ccccctcggg atctcctctc tctctgcaga agggagagct atttctcttt 1200

tttcttaatt tcgcctatta aacctccatt cttaatcttc actccacgtg tgtccgtgtc 1260

cttgatttcc ttggcatgag gcaaccaacc tcaggtatta ccccagacaa acgatgccgc 1320

ttcatgggga ctcctcacct gggatgtstg atcctagaat tgcaggagtg agcagactca 1380

gggtagttat tttccccatc tgtatttggt tcatgctaac tgatgcctcc actttaacag 1440

gcaagccctg ccccatcctt tccctgttag cctgtgggac tctcgtgaga acacagatac 1500

gtgtgcagct acagaaccac ccttttcctt gctctcttgt catttact 1548

<210> 14
<211> 1001
<212> DNA
<213> Homo sapiens

<220>

<221> source
<222> 1..1001
<223> /mol_type="DNA"
      /note="|rs11031746"
      /organism="Homo sapiens"

<220>
<221> misc_difference
<222> 501
<223> /allele="alleles='A/C'"

<400> 14
cttggcctcc caaagtgcgg ggattacagg cgtgagccac tgcacccaga tgagattgag      60

acttagattt aggactgtca ggggtcggca gttggctgga tgttgggtga ggattacaga     120

aggaggagtc ccaggtttct ggcttgagct acatggggaa gaaggccatg gttcctgttt     180

tgaacatgtt gtcttggtgc ctaggagaga tcggagtgga ctgcctagga aacagctggc     240

tgcagctctg tcttggggtc tggtgaggat taaacaaatg aagtatggaa agcacttagc     300

ccagtggctg gcacatagtc cgtacttaat aaatatgaaa tattattatt atgtattagt     360

tttatattca ggtctgtgct gtaccttatc aaattatgcc ctgagagcca tttggagcaa     420

aataaaaaga aggcaagtaa atgacaagag agcaaggaaa agggtggttc tgtagctgca     480

cacgtatctg tgttctcacg mgagtcccac aggctaacag ggaaaggatg gggcagggct     540

tgcctgttaa agtggaggca tcagttagca tgaaccaaat acagatgggg aaaataacta     600

ccctgagtct gctcactcct gcaattctag gatcagacat cccaggtgag gagtccccat     660

gaagcggcat cgtttgtctg gggtaatacc tgaggttggt tgcctcatgc caaggaaatc     720

aaggacacgg acacacgtgg agtgaagatt aagaatggag gtttaatagg cgaaattaag     780

aaaaaagaga aatagctctc ccttctgcag agagagagga gatcccgagg gggtcttctg     840

gtttggtggt gaaatgcaca gggttttata gtctggtttg cggaggctgt gtctgactta     900

catagggccc aaagattggt tggaccaggc atgacattta tacagcccgt gaagaagttg     960

gccaccccac cctaatcttt tgttatgcaa atgagttttt t                        1001

<210> 15
<211> 1153
<212> DNA
<213> Homo sapiens

<220>
<221> source
<222> 1..1153
<223> /mol_type="DNA"
      /note="rs11031745"
      /organism="Homo sapiens"

<220>
<221> misc_difference
<222> 953
<223> /allele="alleles='C/T'"

<400> 15

atgaagtaga tctttggagc tgggagtcac tgagcaagtt gttacaaggc tgcaaggtaa 60

ggaccaggca tgaaaggcct gggaagccct gggaagttta ggctttaacc taagggcaaa 120

gggtggcaag gtgagatgga aaagtcactt ggctcctatg ccagtccaga ttgaagggct 180

ggtgatggag agaatagcca ggaggtatga tagtaatcca gagacagatg ctgggactag 240

gagggctggg gacaggtgga ggcatgagga gagattgaga tttatttatt tgttttatta 300

ttattattct ttgagacaca gtctccctct gtcgcccagg ctggagtgca gtggcactat 360

ctcagctcac tgcaacctct gcctccctgg ttggagcaat tcttatgcct cagcctcctg 420

agtaatcctc agctgggatt acaggcgtgc gtcaccatgc ccggctaatt tttttgtatt 480

tttaatagag atggggtttc accatgtttc ccaggctggt ctcaaactcc tgggctcaag 540

tgatccacct gccttggcct cccaaagtgc ggggattaca ggcgtgagcc actgcaccca 600

gatgagattg agacttagat ttaggactgt caggggtcgg cagttggctg gatgttgggt 660

gaggattaca gaaggaggag tcccaggttt ctggcttgag ctacatgggg aagaaggcca 720

tggttcctgt tttgaacatg ttgtcttggt gcctaggaga gatcggagtg gactgcctag 780

gaaacagctg gctgcagctc tgtcttgggg tctggtgagg attaaacaaa tgaagtatgg 840

aaagcactta gcccagtggc tggcacatag tccgtactta ataaatatga aatattatta 900

ttatgtatta gttttatatt caggtctgtg ctgtacctta tcaaattatg ccytgagagc 960

catttggagc aaaataaaaa gaaggcaagt aaatgacaag agagcaagga aaagggtggt 1020

tctgtagctg cacacgtatc tgtgttctca cgagagtccc acaggctaac agggaaagga 1080

tggggcaggg cttgcctgtt aaagtggagg catcagttag catgaaccaa atacagatgg 1140

ggaaaataac tac 1153

<210> 16
<211> 1105
<212> DNA
<213> Homo sapiens

<220>
<221> source
<222> 1..1105
<223> /mol_type="DNA"
/note="rs11031744"
/organism="Homo sapiens"

<220>
<221> misc_difference
<222> 905
<223> /allele="|alleles='C/T'"

<400> 16
agtttagaac aaaggcttaa aagtcacaga gaatgaagta gatctttgga gctgggagtc 60

actgagcaag ttgttacaag gctgcaaggt aaggaccagg catgaaaggc ctgggaagcc 120

ctgggaagtt taggctttaa cctaagggca aagggtggca aggtgagatg gaaaagtcac 180

ttggctccta tgccagtcca gattgaaggg ctggtgatgg agagaatagc caggaggtat 240

gatagtaatc cagagacaga tgctgggact aggagggctg gggacaggtg gaggcatgag 300

gagagattga gatttattta tttgttttat tattattatt ctttgagaca cagtctccct 360

ctgtcgccca ggctggagtg cagtggcact atctcagctc actgcaacct ctgcctccct 420

ggttggagca attcttatgc ctcagcctcc tgagtaatcc tcagctggga ttacaggcgt 480

gcgtcaccat gcccggctaa tttttttgta tttttaatag agatggggtt tcaccatgtt 540

tcccaggctg gtctcaaact cctgggctca agtgatccac ctgccttggc ctcccaaagt 600

gcggggatta caggcgtgag ccactgcacc cagatgagat tgagacttag atttaggact 660

gtcaggggtc ggcagttggc tggatgttgg gtgaggatta cagaaggagg agtcccaggt 720

ttctggcttg agctacatgg ggaagaaggc catggttcct gttttgaaca tgttgtcttg 780

gtgcctagga gagatcggag tggactgcct aggaaacagc tggctgcagc tctgtcttgg 840

ggtctggtga ggattaaaca aatgaagtat ggaaagcact tagcccagtg gctggcacat 900

agtcygtact taataaatat gaaatattat tattatgtat tagttttata ttcaggtctg 960

tgctgtacct tatcaaatta tgccctgaga gccatttgga gcaaaataaa aagaaggcaa 1020

gtaaatgaca agagagcaag gaaaagggtg gttctgtagc tgcacacgta tctgtgttct 1080

cacgagagtc ccacaggcta acagg 1105

<210> 17
<211> 1053
<212> DNA
<213> Homo sapiens

<220>
<221> source
<222> 1..1053
<223> /mol_type="DNA"
/note="rs3858436"
/organism="Homo sapiens"

<220>
<221> misc_difference
<222> 796
<223> /allele="alleles='A/G'"

<400> 17
tgctctcttg tcatttactt gccttctttt tattttgctc caaatggctc tcagggcata 60

atttgataag gtacagcaca gacctgaata taaaactaat acataataat aatatttcat 120

atttattaag tacggactat gtgccagcca ctgggctaag tgctttccat acttcatttg 180

tttaatcctc accagacccc aagacagagc tgcagccagc tgtttcctag gcagtccact 240

ccgatctctc ctaggcacca agacaacatg ttcaaaacag gaaccatggc cttcttcccc 300

atgtagctca agccagaaac ctgggactcc tccttctgta atcctcaccc aacatccagc 360

caactgccga cccctgacag tcctaaatct aagtctcaat ctcatctggg tgcagtggct 420

cacgcctgta atccccgcac tttgggaggc caaggcaggt ggatcacttg agcccaggag 480

tttgagacca gcctgggaaa catggtgaaa ccccatctct attaaaaata caaaaaaatt 540

agccgggcat ggtgacgcac gcctgtaatc ccagctgagg attactcagg aggctgaggc 600

ataagaattg ctccaaccag ggaggcagag gttgcagtga gctgagatag tgccactgca 660

ctccagcctg ggcgacagag ggagactgtg tctcaaagaa taataataat aaaacaaata 720

aataaatctc aatctctcct catgcctcca cctgtcccca gccctcctag tcccagcatc 780

tgtctctgga ttactrtcat acctcctggc tattctctcc atcaccagcc cttcaatctg 840

gactggcata ggagccaagt gacttttcca tctcaccttg ccaccctttg cccttaggtt 900

aaagcctaaa cttcccaggg cttcccaggc ctttcatgcc tggtccttac cttgcagcct 960

tgtaacaact tgctcagtga ctcccagctc caaagatcta cttcattctc tgtgactttt 1020

aagcctttgt tctaaactat ctgaattctt tca 1053

<210> 18
<211> 1410
<212> DNA
<213> Homo sapiens

<220>
<221> source
<222> 1..1410
<223> /mol_type="DNA"
/note="rs2418901"
/organism="Homo sapiens"

<220>
<221> misc_difference
<222> 886
<223> /allele="alleles='A/G'"

<400> 18
ataataactc aaagagaggg ccgggcacgg tggctcacac ctgtaatccc agtactttgg 60

gaggccaagg caggtggatc acaaggtcaa gagatcaata ccgtcctggc caacatgctt 120

aaaccccatc cctactaaaa atacaaaaat tagccaggtg tggtggcatg cacctgtaat 180

cccaactact caggaggctg aggcaggaga atcgcttgaa cctgggaggt ggagattgta 240

gtgagcccag ttcgcaccac tgcactccaa cctggcaaca gagcgagact ccatcaaaac 300

aacaacaaca acaacaaaac aaagcaaaaa agcaaaaaca aaacaaacaa aaaagaaact 360

tcaaagagag accagctggc catttcttgt aatcacacca ctccactgag gaagagaacc 420

ccaggactca ctgctagagt agggaggtgg ggagcaaaat gctaggagaa aaagagaggt 480

taatatttca aagatggtac cctatcgcat attattgagc acatgctatg gagcagtcac 540

tgttctggat gctaaggata tagagatgga tgcaatctac acaggggatg aatttgtttc 600

caattgttgc tataaaaaat taccttgtgg attaaaacaa cacaaatttc ttatagtttt 660

ggagatcagc agtccaaaat ggttctcact gggctaaaat cgaggtatca acagggccac 720

tttcctttcg gaagatctag gggatgatgc attttcacac cttctccagc tgcccacatt 780

tcttggctcg tggcctgtat cactccgagc tttgcttcct ccatcacatc tccttctctg 840

atcctcatgc tcctgctccc cactctctgc tttataagga cccttrttac atttagagtc 900

cacctccaga atccaggaaa atctcccaat cttaaaatcc ttagcttaat cacatcagca 960

aagtcccctt tgccttgtat cctgggttct agggattagg acatggacat ctgtgggaag 1020

acattaatct gcctactaca ggggatttgt ctagaggtgc tgtggtaagc cctaccacag 1080

gagaaatgta gggtgtatga gagtttgcag gaatgccaga tttggcgatc aggaaaagct 1140

cctctgttta gtctgagacc tggaggatga ataagaagga tttgggaggc cgggtgtggt 1200

ggctcatgct tgtaatccca gctacttggg aggctgaggc aggagaatca cttgaaccca 1260

agaggtgagg ttgcagtgag ctgagatcga gccactgcat tccagcctgg gcgacagagc 1320

aagcctctgc ctcaaaaaaa aaaaaaaaaa aaaaagaagg atttgggaga catgaggaag 1380

aggaagttat actgaaagaa ttcagatagt 1410